(19) **Europäisches Patentamt / European Patent Office / Office européen des brevets**

(11) **EP 1 578 457 B1**

(12) **EUROPEAN PATENT SPECIFICATION**

(45) Date of publication and mention
of the grant of the patent:
**12.09.2007 Bulletin 2007/37**

(51) Int Cl.:
*A61L 9/01* (2006.01)      *B01J 20/02* (2006.01)
*B01J 23/04* (2006.01)     *B01J 23/70* (2006.01)
*A61L 9/012* (2006.01)

(21) Application number: **03813982.0**

(22) Date of filing: **11.06.2003**

(86) International application number:
**PCT/KR2003/001149**

(87) International publication number:
**WO 2004/058312 (15.07.2004 Gazette 2004/29)**

(54) **CARBON NANOBALL FOR DEODORIZATION**

NANO-KOHLENSTOFFKUGEL ZUR DESODORIERUNG

NANOBILLE DE CARBONE A ACTION DESODORISANTE

(84) Designated Contracting States:
**AT BE BG CH CY CZ DE DK EE ES FI FR GB GR
HU IE IT LI LU MC NL PT RO SE SI SK TR**

(30) Priority: **27.12.2002 KR 2002084983
28.12.2002 KR 2002085851
28.12.2002 KR 2002085852**

(43) Date of publication of application:
**28.09.2005 Bulletin 2005/39**

(73) Proprietor: **LG Household & Health Care Ltd.
Seoul 150-721 (KR)**

(72) Inventors:
• **SONG, Jun-Yeob**
**Suwon-si
440-709 Gyeonggi-do (KR)**
• **KIM, Jong-Yun**
**305-728 Daejeon (KR)**
• **PARK, Seung-Kyu**
**305-728 Daejeon (KR)**
• **YU, Jong-Sung**
**302-776 Daejeon (KR)**
• **PARK, Yong-Ki**
**305-755 Daejeon (KR)**
• **LEE, Chul-Wee**
**305-345 Daejeon (KR)**

• **KANG, Yun-Seog**
**305-761 Daejeon (KR)**

(74) Representative: **Hinkelmann, Klaus et al
Stolmár Scheele Hinkelmann
Blumenstrasse 17
80331 München (DE)**

(56) References cited:
**EP-A- 0 893 128          EP-A- 1 300 364
WO-A-01/89991          WO-A-02/38520
WO-A-03/006372          JP-A- 2001 172 089
KR-B1- 100 292 140**

• **PATENT ABSTRACTS OF JAPAN vol. 2002, no. 03, 3 April 2002 (2002-04-03) & JP 2001 321424 A (DENSO CORP), 20 November 2001 (2001-11-20)**
• **LEE J ET AL: "DEVELOPMENT OF A NEW MESOPOROUS CARBON USING AN HMS ALUMINOSILICATE TEMPLATE" ADVANCED MATERIALS, WILEY VCH, WEINHEIM, DE, vol. 12, no. 5, 2 March 2000 (2000-03-02), pages 359-362, XP000919780 ISSN: 0935-9648**
• **YOON S.B. ET AL.: 'FABRICATION OF CARBON CAPSULES WITH HOLLOW MACROPOROUS CORE/MESOPOROUS SHELL STRUCTURES' ADV. MATER. vol. 14, no. 1, January 2002, pages 19 - 21, XP001129559**

**Description**

TECHNICAL FIELD

The present invention relates to a carbon nanoball for deodorization, and more particularly to a metal-impregnated carbon nanoball which is composed of a porous carbon shell having a spherical hollow core and used for deodorization.

BACKGROUND ART

Generally, various bad smells are generated from daily necessaries such as refrigerator, air conditioner, diaper, hygienic band, cigarette, footwear cabinet and clothes chest and in daily life area such as bedroom, bathroom and automobile room. In addition, bad smells are also generated from exhaust gas of automobiles and industrial equipments such as refuse disposal plant, wastewater disposal plant and factories. Materials generating bad smells are representatively as follows: methanthiol, methyl sulfide, dimethyl disulfide, hydrogensulfide, ammonia, trimethylamine, acetaldehyde, nitric oxide, nitrous oxide, styrene and so on.

Also, various kinds of deodorizing agents have been developed in order to eliminate such bad smells.

Recently, a method for making a carbon nanoball composed of a porous carbon shell having a spherical hollow core (Adv. Mater. 2002, 14, no. 1, January 4) is proposed. This carbon nanoball has the advantage that it may adsorb more various kinds of malodor substances than a conventional activated carbon deodorizing agent. However, the carbon nanoball has some limitations that it may not adsorb any more malodor substances after adsorbing a certain amount. In addition, the above-mentioned carbon nanoball is a limited capacity in deodorizing.

DISCLOSURE OF INVENTION

The present invention is designed to solve such drawbacks of the prior art, and therefore an object of, the present invention is to provide a carbon nanoball having excellent deodorizing ability and capable of adsorbing various kinds of malodor substances.

In order to accomplish the above object, the present invention provides a carbon nanoball for deodorization comprising a porous carbon shell having a spherical hollow core, wherein the spherical hollow core has a diameter of 10-1000 nm and the porous carbon shell has a thickness of 50-500 nm, and at least one metal selected from the group consisting of transition metal, oxidized transition metal and alkali metal salt is impregnated to the shell.

Preferably, the transition metal may be selected from the group consisting of Copper (Cu), Iron (Fe), Manganese (Mn), Nickel (Ni), Cobalt (Co), Silver (Ag), Gold (Au), Vanadium (V), Ruthenium (Ru), Titanium (Ti), Chrome (Cr), Zinc (Zn) and Palladium (Pd), and the alkali metal salt may be selected from the group consisting of sodium bromide (NaBr), sodium iodide (NaI), potassium bromide (KBr), potassium iodide (KI) and potassium iodate ($KIO_3$).

BRIEF DESCRIPTION OF THE DRAWINGS

These and other features, aspects, and advantages of preferred embodiments of the present invention will be more fully described in the following detailed description, taken accompanying drawings. In the drawings:

FIG. 1 is a schematic diagram for illustrating the process for making a carbon nanoball for deodorization according to the present invention; and

FIGs. 2 and 3 are graphs showing deodorizing effects of the carbon nanoball for deodorization according to the present invention to ammonia and methanthiol.

BEST MODES FOR CARRYING OUT THE INVENTION

Hereinafter, embodiments of the present invention will be described, however the present invention is not limited to the following embodiments but capable of being modified in diverse ways within the scope of the invention.

A carbon nanoball of the present invention has a ball-shaped carbon structure composed of a hollow core and a porous shell. To the shell, transition metal, oxidized transition metal, alkali metal salt or their mixture is impregnated. The mesoporous pores formed in the shell may not only adsorb various kinds of malodor substances but also chemically adsorb and destroy the malodor substances for deodorization. This carbon nanoball for deodorization of the present invention shows more excellent deodorizing ability than the impregnated activated carbon disclosed in Korean Laid-open Patent Publication No. 1999-80808. In other words, since the activated carbon has microporous pores, the pores may be clogged and deteriorate the deodorizing ability when the deodorizing materials are impregnated. However, since the carbon nanoball for deodorization of the present invention has the mesoporous pores in the shell, such a problem is not caused. In addition, since the malodor substances are captured in the hollow core of the carbon nanoball for deodorization of the present invention, differently to the impregnated activated carbon, it is possible to give sufficient contact time between the malodor substances and the deodorizing material impregnated on the inner surface of the shell. In addition, the carbon nanoball of the present invention may prevent secondary pollution caused when decomposition products generated by the deodorizing materials are emitted outside.

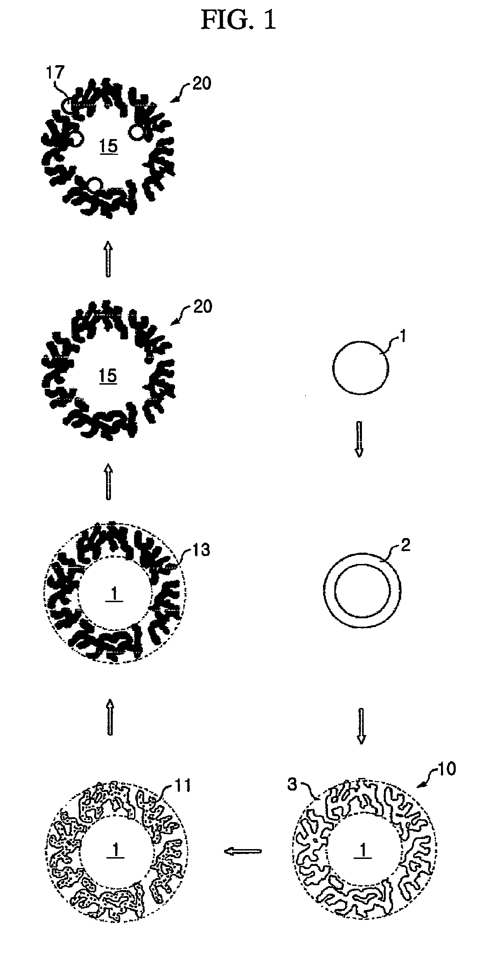

An example of a method for making the carbon nanoball for deodorization according to the present invention is described in detail with reference to FIG. 1.

At first, a spherical silica core 1 is prepared. The silica core 1 may be composed according to the well-known Stober method (Stober, W.; Fink, A.; Bohn, E. J. Colloid Inter. Sci.1968, 26, 62) from a silica precursor such as tetramethylorthosilicate and tetraethylorthosilicate. The silica core has a diameter of 10 ~ 1,000nm.

After that, a shell 2 is grown up on the surface of the silica core 1 by using silica precursor and surface active agent such as alkyltrimethoxysilane expressed as the following Chemical Formula 1, alkyltriethoxysilane expressed as the following Chemical Formula 2, halogenated alkyltrimethylammonium expressed as the following Chemical Formula 3, alkylpolyoxyethylene expressed as the following Chemical Formula 4 and glycerolethoxylate expressed as the following Chemical Formula 5, in a solvent.

$$\text{Chemical Formula 1} \qquad R_1R_2R_3Si(OCH_3)_3$$

In the Chemical Formula 1, $R_1$, $R_2$ and $R_3$ are methyl groups, and $R_4$ is an alkyl group having a carbon number of 12 to 22.

$$\text{Chemical Formula 2} \qquad R_1R_2R_3R_4Si(OC_2H_5)_3$$

In the Chemical Formula 2, $R_1$, $R_2$ and $R_3$ are ethyl groups, and $R_4$ is an alkyl group having a carbon number of 12 to 22.

$$\text{Chemical Formula 3} \qquad R_1R_2R_3R_4NX$$

In the Chemical Formula 3, $R_1$, $R_2$ and $R_3$ are independently methyl or ethyl groups, $R_4$ is an alkyl group having a carbon number of 12 to 22, and X is halogen.

$$\text{Chemical Formula 4} \qquad R(OCH_2CH_2)nOH$$

In the Chemical Formula 4, R is an alkyl group having a carbon number of 12 to 22, and n is an integer in the range of 3 ~ 20.

$$\text{Chemical Formula 5} \qquad CH_2(CH_2O)n_1HCH(CH_2O)_{n2}HCH_2(CH_2O)n_3H$$

In the Chemical Formula 5, $n_1$, $n_2$ and $n_3$ are independently integers in the range of 4 ~ 20.

And then, after the products having the shell is selectively filtered and calcined at, for example, 500 ~ 600°C to remove the surface active agent components. Then, particles 10 having a silica shell 3 are obtained in which mesoporous pores having a certain size are formed in the place where the surface active agent is removed. The size of the mesoporous pore and the thickness of the shell may be controlled by changing the kind of the surface active agent and the kind and molecular ratio of the silica precursor. The porous carbon shell has a thickness of 50 ~ 500nm.

Subsequently, a monomer 11, such as acrylonitrile, phenol-formaldehyde and divinylbenzene, which is capable of forming polymer, is injected into the mesoporous pores formed in the shell. After that, the monomer is polymerized to form a carbon precursor and the particle 10 having the silica shell is formed. Preferably, the monomer is polymerized using the condensation polymerization or radical polymerization. In addition, in the radical polymerization, the monomer is sufficiently mixed with the radical initiator and the injected into the mesoporous pores of the silica particle, and then polymerized according to the characteristics of the monomer. At this time, as for a radical initiator, for example, azobisisobutyronitrile (AIBN), t-butyl peracetate, benzoyl peroxide, acetyl peroxide and lauryl peroxide may be used. This polymerization is well known in the art, and preferably conducted for about 12 hours at 60 ~ 130°C to make the silica structure containing polymer.

And then, the treatment of the silica structure containing the polymer (or, carbon precursor) under the nitrogen atmosphere at about 1,000°C makes the silica structure contain a carbonized polymer 13. After that, a carbon nanoball 20 having a ball shape which has a spherical hollow core 15 and a porous carbon shell is obtained as a consequence of etching the carbonized silica structure by hydrofluoric acid solution or sodium hydroxide/ethyl alcohol mixed solution.

After that, the carbon nanoball 20 is dipped into an aqueous solution composed of transition metal, oxidized transition metal, alkali metal salt or their mixture and matured at the room temperature for 2 ~ 3 days, and then filtered and dried at 70 ~ 110°C to make the metal-impregnated carbon nanoball according to the present invention. As for the transition metal or the oxidized transition metal which may be impregnated to the shell, Copper (Cu), Iron (Fe), Manganese (Mn), Nickel (Ni), Cobalt (Co), Silver (Ag), Gold (Au), Vanadium (V), Ruthenium (Ru), Titanium (Ti), Chrome U (Cr), Zinc (Zn), Palladium (Pd) or their oxide may be used. As for the alkali metal, sodium bromide (NaBr), sodium iodide (NaI), potassium bromide (KBr), potassium iodide (KI) and potassium iodate ($KIO_3$) may also be used. An impregnated amount of the metal may be controlled by changing the concentration of the metal-containing aqueous solution or the infiltration time, and is preferably 0.01 - 30 wt% on the basis of the total weight of the carbon nanoball for deodorization.

The metal-impregnated carbon nanoball according to the present invention may be provided with one or more kinds of metals among the above-mentioned metals. Thus, the deodorizing agent containing the metal-impregnated carbon nanoball according to the present invention may be prepared or composed in various ways depending on the kind of bad smell or its usage. For example, a deodorizing agent may contain carbon nanoball to which only one kind of metal is impregnated, or to which two different kinds of metals are impregnated, or more than two kinds of metals are impregnated.

The metal-impregnated carbon nanoball of the present invention may be used for deodorizing and eliminating various odor materials such as methanthiol, methyl sulfide, dimethyl disulfide, hydrogen sulfide, ammonia, trimethylamine, styrene, acetaldehyde, nitric oxide, nitrous oxide, indoor bad smells generated in bathroom, kitchen or footwear cabinet in home, and smell of tobacco. Thus it also may give excellent effects in eliminating bad smells of refrigerator, air conditioner, air cleaner, automobile room, exhaust gas of cars as well as a human body.

In addition, the metal-impregnated carbon nanoball of the present invention may be uniformly dispersed and stuck to one having a shape of sheet, pack or pad, thus it may be applied to goods such as a diaper for the infant or the person suffered from the incontinence or a hygienic band for women which use such matters.

## Embodiment 1 to 4

A spherical silica core is composed according to the well-known Stober method by using tetraethoxysilane as a silica precursor. At this time, tetraethoxysilane is put therein and reacted together with octadecyltrimethoxysilane ($C_{18}$-TMS), and then filtered to obtain silica particles. The silica particles are thermally treated at 550°C for 5 hours so that mesoporous pores having a certain size are formed in the place where the surface active agent is removed. Then, divinylbenzene is sufficiently mixed with azobisisobutyronitrile (AIBN), which is a radical initiator, and the injected into the mesoporous pores of the silica particle, and then polymerized at 130°C for about 12 hours to make a silica structure containing polymer. In succession, the silica structure containing polymer is carbonized under nitrogen circumstance at 1,000°C to form a carbon/silica composite. Subsequently, the carbon/silica composite is put into hydrofluoric acid to remove inorganic structure of the carbon/silica composite, therefore the carbon nanoball which has a ball-shaped carbon structure including a hollow core and a porous shell is obtained.

After that, in order to impregnate the metals of the following Table 1, the carbon nanoball made along with the above-mentioned method is dipped into the 1N of metal solution for about 50 hours, filtered and dried at 70°C, therefore a metal-impregnated carbon nanoball is obtained.

Among the metal-impregnated carbon nanoball A ~ H, 0.01g of A, D, E and H are picked as Embodiments 1 ~ 4, respectively.

Table 1

|  | The kind of impregnated metal (impregnated amount of metal, %) |
|---|---|
| A | copper (1.3) + manganese (0.3) |
| B | nickel (3.1) + iron (0.8) |
| C | gold (0.8) + chrome (0.9) + palladium (0.8) |
| D | copper (3.1) + iron (0.8) + zinc (0.8) |
| E | potassium iodide (3.4) |
| F | silver (4.2) |
| G | cobalt (2.1) + potassium iodate (1.3) |
| H | vanadium (2.1) + ruthenium (0.3) + titanium (0.6) |

In Table 1, Impregnated Amount (%) of Metal = Weight of Metal/Weight of Carbon nanoball x 100.

### Comparative Example 1

A carbon nanoball to which no metal is impregnated is made in the same way of the above embodiments, and 0.01g is taken from the carbon nanoball as Comparative Example 1.

### Comparative Example 2

A carbon nanoball to which no metal is impregnated is made in the same way of the above embodiments, and 0.1g is taken from the carbon nanoball as Comparative Example 2.

### Comparative Example 3

0.1g of activated carbon (manufactured by Junsei in Japan) is taken as Comparative Example 3.

### Comparative Examples 4 ~ 8

0.1g of various commercially-used conventional deodorizers are taken as Comparative Examples 4 ~ 8.

### Comparative Examples 9 ~ 12

4 kinds of metal-impregnated activated carbons made by impregnating metals to the activated carbon (manufactured by Junsei in Japan) with same metal compositions as Embodiments 1 to 4 are respectively taken as Comparative Examples 9 ~ 12.

In the estimation of the deodorizing effects, trimethylamine, ammonia, methanthiol and acetaldehyde are used as an odor source. At first, deodorizing agents having a weight shown in Table 2 are respectively put into a 250ml transparent container which contains malodor substances such as 0.2ml of ammonia 0.1% solution, 0.07ml of trimethylamine 1% solution, 0.15ml of acetaldehyde 1% solution and 0.12ml of methanthiol 0.1% benzene solution. After that, the container is sealed with a cap in which a detecting tube for measuring a residual amount of the malodor substances is attached, and left alone for 30 minutes. After that, with passing the gas in the container through the detecting tube, the color change of the detecting tube is monitored to measure the capability of the deodorizing agent.

This capability (%) of the deodorizing agent is calculated according to the following formula on the basis of the blank test in which only the odor source is put without the deodorizing agent, and the calculated results are shown in Table 2.

$$\text{Deodorizing Capability (\%)} = [(\text{Detecting Tube Value of Blank Test (ppm)} - \text{Measured Detecting Tube Value (ppm)})/(\text{Detecting Tube Value of Blank Test (ppm)})] \times 100$$

Table 2

| | Deodorizing agent (weight) | Deodorizing capability (%) | | | |
|---|---|---|---|---|---|
| | | Ammonia | Trimethylamine | Acetaldehyde | Methanthiol |
| Embodiment 1 | Metal-impregnated carbon nanoball (0.01g) | 78 | 87 | 63 | 90 |
| Embodiment 2 | Metal-impregnated carbon nanoball (0.01g) | 98 | 94 | 47 | 93 |
| Embodiment 3 | Metal-impregnated carbon nanoball (0.01g) | 89 | 92 | 54 | 98 |

(continued)

|  | Deodorizing agent (weight) | Deodorizing capability (%) | | | |
|---|---|---|---|---|---|
|  |  | Ammonia | Trimethylamine | Acetaldehyde | Methanthiol |
| Embodiment 4 | Metal-impregnated carbon nanoball (0.01g) | 88 | 86 | 58 | 92 |
| Comparative example 1 | Carbon nanoball (0.01g) | 43 | 85 | 7 | 29 |
| Comparative example 2 | Carbon nanoball (0.1g) | 80 | 84 | 40 | 91 |
| Comparative example 3 | Activated carbon (0.1g) | 61 | 58 | 20 | 48 |
| Comparative example 4 | β-Cyclodextrin (0.1g) | 15 | 2 | 0 | 0 |
| Comparative example 5 | Copper chloride (98%, 0.1g) | 68 | 85 | 4 | 84 |
| Comparative example 6 | Carbazole W7 MCT (0.1g) | 60 | 9 | 12 | 0 |
| Comparative example 7 | Aluminum chloride (0.1g) | 92 | 97 | 0 | 0 |
| Comparative example 8 | Zeolites (0.1g) | 30 | 27 | 8 | 0 |
| Comparative example 9 | Metal-impregnated activated carbon (0.01g) | 76 | 67 | 34 | 68 |
| Comparative example 10 | Metal-impregnated activated carbon (0.01g) | 78 | 58 | 40 | 64 |
| Comparative example 11 | Metal-impregnated activated carbon (0.01g) | 69 | 66 | 47 | 72 |
| Comparative example 12 | Metal-impregnated activated carbon (0.01g) | 70 | 69 | 45 | 70 |

From Table 2, it will be understood that the carbon nanoball for deodorization according to the embodiments of the present invention shows excellent deodorizing effects against 4 kinds of odorizing sources, compared with the comparative examples, even though a small amount as much as 10% (0.01g) is used. In addition, it will be also understood from Table 2 that the deodorizing effects of the metal-impregnated carbon nanoball increase about 20% or above for the four odorizing sources.

**Embodiment 5**

4 types of metal-impregnated carbon nanoballs are made according to the same way as the Embodiment 1 with the metals shown in the following Table 3, respectively. Then, deodorizing filter for refrigerator is prepared by mixing the 4 kinds of metal-impregnated carbon nanoball with a polypropylene polymer binder as much as 20 wt% of the total filter weight, respectively and executing further treatment. Among the metal-impregnated carbon nanoballs I~K, K is taken as Embodiment 5.

**Comparative Example 13**

The Comparative Example 13 is identical to Embodiment 5, except that an metal-impregnated activated carbon made by impregnating the metals to the activated carbon (manufactured by Junsei in Japan) is used instead of the carbon nanoball of Embodiment 5.

Table 3

|  | Kind of impregnated metal (impregnated amount of metal, %) |
|---|---|
| I | copper(1.3)+manganese(0.3) |
| J | cobalt(2.1)+iron(0.8)+zinc(0.3) |

(continued)

| | Kind of impregnated metal (impregnated amount of metal, %) |
|---|---|
| K | potassium iodide(3.2)+copper(0.8) |

In Table 3, Impregnated Amount of Metal (%) = Weight of Metal/Weight of Carbon nanoball x 100

The test of deodorizing effects is conducted under the experimental condition as shown in the following Table 4.

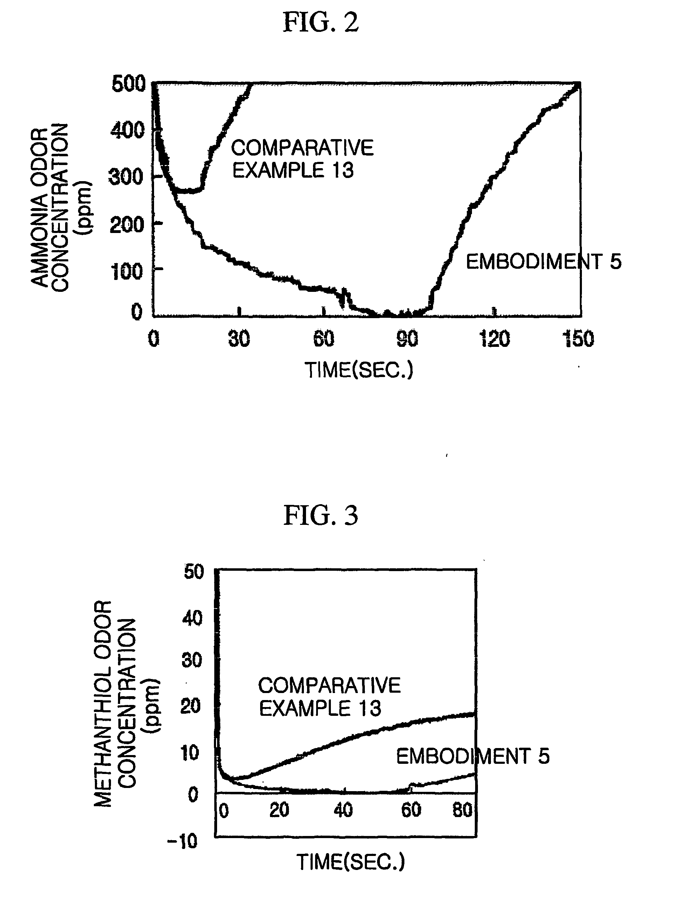

In case of the deodorizing effect test for ammonia, 0.5g of the deodorizing filters of Embodiment 5 and Comparative Example 13 are put into tube reactors, respectively. After that, the ammonia gas is flowed through each tube reactor, and then the concentrations of the discharged gas are analyzed, of which results are expressed in Table 5 and FIG. 2.

In case of the deodorizing effect test for methanthiol, 0.01g of the deodorizing agents of Embodiment 5 and Comparative Example 13 are put into tube reactors, respectively. After that, the methanthiol gas is flowed through each tube reactor, and then the concentrations of the discharged gas are analyzed, of which results are expressed in Table 5 and FIG. 3.

At this time, a gas chromatograph or a mass spectrometer is used as an analyzer, and the relative humidity is 50%.

Table 4

| Odor sources | Adsorption temperature (°C) | Relative humidity (RH) | Gas flow rate (cc/min) | Amount of adsorbent (g) | Initial gas concentration (ppm) |
|---|---|---|---|---|---|
| Ammonia | Room temperature | 50% | 600 | 0.5 | 500 |
| Methanthiol | Room temperature | 50% | 100 | 0.01 | 50 |

Here, in order to analyze the odor gas concentration, the concentrations of the discharged gas of the comparative example and the embodiment are comparatively measured. The dynamic test was carried out to evaluate the capacity of carbon for removal of odor substances, based on ASTM D28 as a standard test method. Break-point is defined as breakthrough time in the breakthrough curve.

Table 5

| | Deodorizing agent (weight) | Odor source | Deodorizing effect | |
|---|---|---|---|---|
| | | | Break-point (min.) | Concentration at break-point (ppm) |
| Embodiment 5 | Metal-impregnated carbon nanoball (0.5g) | Ammonia | 80 ~ 90 | 0 |
| Comparative example 13 | Metal-impregnated activated carbon (0.5g) | Ammonia | 8 ~ 16 | 260 |
| Embodiment 5 | Metal-impreganted carbon nanoball (0.01g) | Methanthiol | 50 ~ 55 | 0 |
| Comparative example 13 | Metal-impregnated activated carbon (0.01g) | Methanthiol | 3 ~ 6 | 3 |

It will be known from Table 5, FIG. 2 and FIG. 3 that the deodorizing agent composition of Embodiment 5 takes a longer time to reach the break-point than the conventional deodorizing agent of Comparative Example 13. In case of ammonia at the break-point, ammonia gas is not detected in Embodiment 5, but is detected to have a concentration of 260 ppm in Comparative Example 13. And in case of methanthiol gas at the break-point, methanthiol gas is not detected in Embodiment 5, but is detected to have a concentration of 3 ppm in Comparative Example 13. Thus, it will be understood from the results that the deodorizing agent of the present invention has much more excellent deodorizing ability in eliminating odors like the ammonia gas and methanthiol than the conventional one.

INDUSTRIAL APPLICABILITY

As described above, the metal-impregnated carbon nanoball according to the present invention adsorbs various kinds of malodor substances as well as shows good deodorizing ability. Thus, the carbon nanoball of the present invention may show excellent deodorizing effect in capturing and resolving the malodor substances when it is used as an deodorizing agent in various odorizing daily necessaries, living spaces, industrial spots and other various stink-generating circumstances.

Claims

1. A carbon nanoball (20) for deodorization comprising a porous carbon shell (13) having a spherical hollow core (15), wherein the spherical hollow core (15) has a diameter of 10 ~ 1,000nm and the porous carbon shell (13) has a thickness of 50 ~ 500nm and at least one metal (17) selected from the group consisting of transition metal, oxidized transition metal and alkali metal salt is impregnated to the shell.

2. A carbon nanoball (20) for deodorization according to claim 1,
   wherein the transition metal is one selected from the group consisting of Copper (Cu), Iron (Fe), Manganese (Mn), Nickel (Ni), Cobalt (Co), Silver (Ag), Gold (Au), Vanadium (V), Ruthenium (Ru), Titanium (Ti), Chrome (Cr), Zinc (Zn) and Palladium (Pd), and
   wherein the alkali metal salt is one selected from the group consisting of sodium bromide (NaBr), sodium iodide (NaI), potassium bromide (KBr), potassium iodide (KI) and potassium iodate ($KIO_3$).

3. A carbon nanoball (20) for deodorization according to claim 1 or 2,
   wherein an impregnated amount of the metal (17) is 0.01 ~ 30 wt% on the basis of the total weight of the carbon nanoball (20) for deodorization.

Patentansprüche

1. Kohlenstoffnanoball (20) zur Desodorierung umfassend eine poröse Kohlenstoffschale (13) mit einem sphärischen hohlen Kern (15), worin der sphärische hohle Kern (15) einen Durchmesser von 10 bis 1000 nm und die poröse Kohlenstoffschale (13) eine Dicke von 50 bis 500 nm aufweist und wobei die Schale mit wenigstens einem Metall (17) ausgewählt aus der Gruppe bestehend aus einem Übergangsmetall, einem oxidierten Übergangsmetall und einem Alkalimetallsalz imprägniert ist.

2. Kohlenstoffnanoball (20) zur Desodorierung gemäß Anspruch 1, worin das Übergangsmetall ausgewählt ist aus der Gruppe bestehend aus Kupfer (Cu), Eisen (Fe), Mangan (Mn), Nickel (Ni), Kobalt (Co), Silber (Ag), Gold (Au), Vanadium (V), Ruthenium (Ru), Titan (Ti), Chrom (Cr), Zink (Zn) und Palladium (Pd) und wobei das Alkalimetallsalz ausgewählt ist aus der Gruppe bestehend aus Natriumbromid (NaBr), Natriumiodid (NaI), Kaliumbromid (KBr), Kaliumiodid (KI) und Kaliumiodat ($KIO_3$).

3. Kohlenstoffnanoball (20) zur Desodorierung gemäß Anspruch 1 oder 2, worin eine imprägnierte Menge des Metalls (17) 0,01 bis 30 Gew.-% bezogen auf das gesamte Gewicht des Kohlenstoffnanoballs (20) für die Desodorierung beträgt.

Revendications

1. Nanobille de carbone (20) à action désodorisante comprenant une enveloppe poreuse de carbone (13) disposant d'un coeur creux sphérique (15), dans laquelle le coeur creux sphérique (15) a un diamètre compris entre 10 et 1000 nm et l'enveloppe poreuse de carbone (13) a une épaisseur comprise entre 50 et 500 nm, et l'enveloppe est imprégnée d'au moins un métal (17) choisi dans le groupe comprenant un métal de transition, un métal de transition oxydé et un sel de métal alcalin.

2. Nanobille de carbone (20) à action désodorisante selon la revendication 1,
   dans laquelle le métal de transition est choisi dans le groupe comprenant le cuivre (Cu), le fer (Fe), le manganèse (Mn), le nickel (Ni), le cobalt (Co), l'argent (Ag), l'or (Au), le vanadium (Va), le ruthénium (Ru), le titane (Ti), le chrome (Cr), le zinc (Zn) et le palladium (Pd), et
dans laquelle le sel de métal alcalin est choisi dans le groupe comprenant le bromure de sodium (NaBr), le iodure de sodium (NaI), le bromure de potassium (KBr), le iodure de potassium (KI) et le iodate de potassium (KIO$_3$).

3. Nanobille de carbone (20) à action désodorisante selon la revendication 1 ou 2, dans laquelle la quantité de métal (17) imprégnée est comprise entre 0,01 et 30% en poids sur la base du poids total de la nanobille de carbone (20) à action désodorisante.

# FIG. 1

## FIG. 2

## FIG. 3

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 199980808

**Non-patent literature cited in the description**

- *Adv. Mater,* 04 January 2002, vol. 14, 1

- **STOBER, W ; FINK, A ; BOHN, E.** *J. Colloid Inter. Sci,* 1968, vol. 26, 62